Europäisches Patentamt

European Patent Office

Office européen des brevets

(11) Publication number: **0 244 148**
**A1**

# (12) EUROPEAN PATENT APPLICATION

(21) Application number: **87303500.0**

(22) Date of filing: **22.04.87**

(51) Int. Cl.4: **C 12 Q 1/06**

(30) Priority: **30.04.86 US 857890**

(43) Date of publication of application:
**04.11.87 Bulletin 87/45**

(84) Designated Contracting States:
**AT BE CH DE FR GB IT LI LU NL SE**

(71) Applicant: **CALGON CORPORATION**
**Route 60-Campbell's Run Road**
**Robinson Township Pennsylvania 15205 (US)**

(72) Inventor: **Farkas, John P.**
**420 Scottsdale Drive**
**Coraopolis Pennsylvania 15108 (US)**

**Jones, Edward H.**
**1427 Adams Street**
**Pittsburgh Pennsylvania 15233 (US)**

(74) Representative: **Hesketh, Alan, Dr.**
**European Patent Department Merck & Co., Inc. Terlings**
**Park Eastwick Road**
**Harlow Essex, CM20 2QR (GB)**

(54) **Method and device for enumerating microorganisms.**

(57) A method and device for enumerating microorganisms in industrial process waters utilizing a dried nutrient broth and an indicator dye system which, when exposed to actively respiring microorganisms, changes color at a rate directly proportional to the number of viable microorganisms present.

**FIG.1**

Vial

Dried Nutrient

Cap

Dried Dye (Inside Cap)

EP 0 244 148 A1

**Description**

"METHOD AND DEVICE FOR ENUMERATING MICROORGANISMS"

Background of the Invention

Excessive biological activity in aqueous systems is indicated by slime deposits, excessive corrosion, pH depression, foul odors or production problems. Biocides are added to aqueous systems to control the level of biological activity and to minimize microbiological-related problems. Since expense precludes an operator from maintaining a sterile environment, a control program must be designed which is cost effective for the system being treated.

Excessive biological activity in certain systems, such as aqueous paper mill systems including stock, white water, filler slurry, coating or starch systems, is especially troublesome. The disadvantage of conventional biological plate counting methods is that papermills generally must take corrective action long before plate counts indicate that a problem exists. For this reason, paper mills are in need of a simple-to-use, rapid and accurate test which quantitatively indicates the level of biological activity.

Conventional biological plate counting methods, which require 48 hours or more to indicate the degree of microbiological contamination, may be of little benefit if a mill system requires immediate treatment. By the time results are obtained from a standard plate count, the mill system may be so fouled as to seriously affect production. Visual inspection of the mill usually will not reveal the existence of a problem until it is too late. Only an actual enumeration of microorganisms will determine if a potential problem exists. The present invention satisfies the need for a rapid, accurate and simple procedure for quantifying microbiological contamination, and therefore is a long-awaited improvement in the art.

To perform a standard plate count, a sample from the system to be tested is serially diluted in sterile, buffered water and aliquots of each dilution are added to a sterile nutrient medium in a petri plate. (See Standard Methods for the Examination of Water and Wastewater, Sixteenth Edition, §907A (1985), which is hereby incorporated into this specification by reference.) Sufficient dilutions are made to accomodate normal paper mill populations which may range from less than 10,000 organisms per ml of sample to more than 100,000,000 organisms per ml. The plates are incubated and individual organisms are allowed to reproduce in the solidified nutrient until distinct, visible colonies of organisms develop by replication of the individual organisms present. A count of the colonies correlates to the number of individual organisms which are present in the original sample. Incubation of bacterial plates generally takes 48 to 72 hours; fungal counts generally require 5 to 7 days.

Practical limitations for use of standard plate counts in industrial plants, such as papermills, include the time required to develop organism colonies and the manpower requirement for setting up plates, sampling, growing colonies and evaluating the results. By the time a problem is recognized, it may be too late to prevent it. For example, some slime-forming bacteria reproduce about every 1/2 hour. This means that their numbers grow expotentially with time. In 1 hour, organism population may multiply 4 times.

An unprotected paper mill system, if contaminated, may continue to run well for 3 to 4 days before odor or production problems develop. Since the standard plate count method usually can detect a biological control problem only after 24 to 48 hours, valuable time is lost, which may result in downtime before the problem is remedied.

Other methods are known in the art for determining microorganism levels. These are discussed below.

    1. Dipsticks are marketed by a number of companies which contain a solid nutrient and an indicator dye which stains individual colonies to facilitate enumeration. The dipstick is momentarily soaked in an aqueous sample. Following incubation of 14 to 48 hours, the number of colonies on the surface is counted, or a comparison is made with a chart to gauge a color change which has been correlated to standard plate counts. This method gives a rough indication of biological activity, and the rapidity of the test is an improvement over the standard plate count method. Unfortunately, the lack of precision and a 24-hour turnaround time is unacceptable in some applications, for example in paper applications.

    2. Adenocine triphosphate (ATP) is a compound commonly associated with biological activity. A luminescence method can be used to quantify the amount of ATP present in a liquid sample. This level, in turn, may be correlated to the level of biological activity at the sample point. However, since different species of bacteria and fungi generate different levels of ATP, a comparison with standard plate counts on a stable population must be performed before the luminescence measure has meaning. This method is therefore most useful for the rapid study of a single specie laboratory culture. Also, since ATP luminescence is fairly complicated and expensive to perform, it is not commonly used as a routine control method.

    3. Methods have also been developed which utilize the color change of an indicator dye, such as 2,3,5-triphenyl tetrozolium chloride (TTC), resazurin, or others. In these methods, either an oxidation-reduction reaction or a PH variation results in a color change in the indicator dye, or the dye is otherwise altered through metabolization by living organisms. Once correlated to standard plate counts, these methods can give semi-quantitative results in hours, although some procedures require up to a day. Unfortunately, complicated, time-consuming sample preparation requirements have made these methods unacceptable for routine control in operating plants.

4. The ninhydrin slime detector spray test is a rapid qualitative amino nitrogen indicator. It is often used in papermills to judge the origin of paper sheet defects. Use of ninhydrin in a quanititative control test method has, so far, been unsuccessful. Ninydrin spray indicates the presence of amino nitrogen which, in turn, is correlated to the slime volume. This test has little value as an early indicator of organism-related problems in industrial systems.

Brief Description of the Drawings

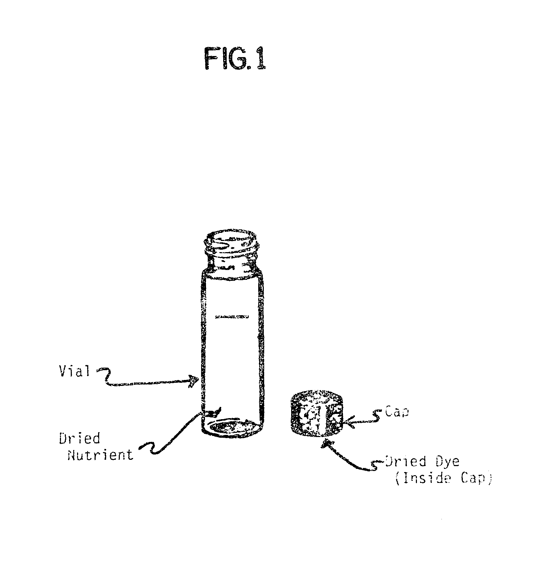

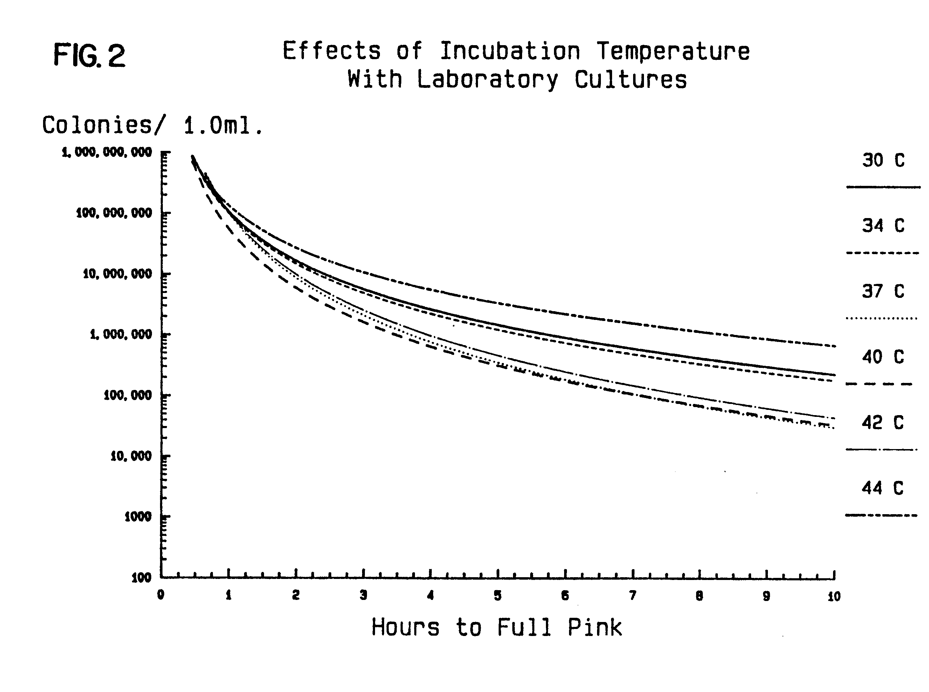

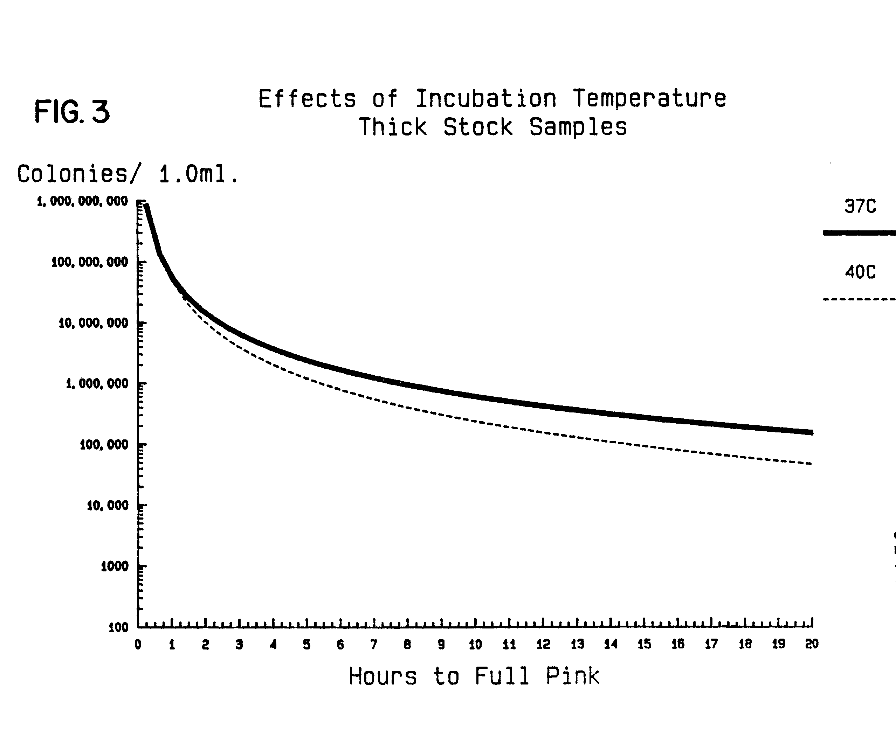

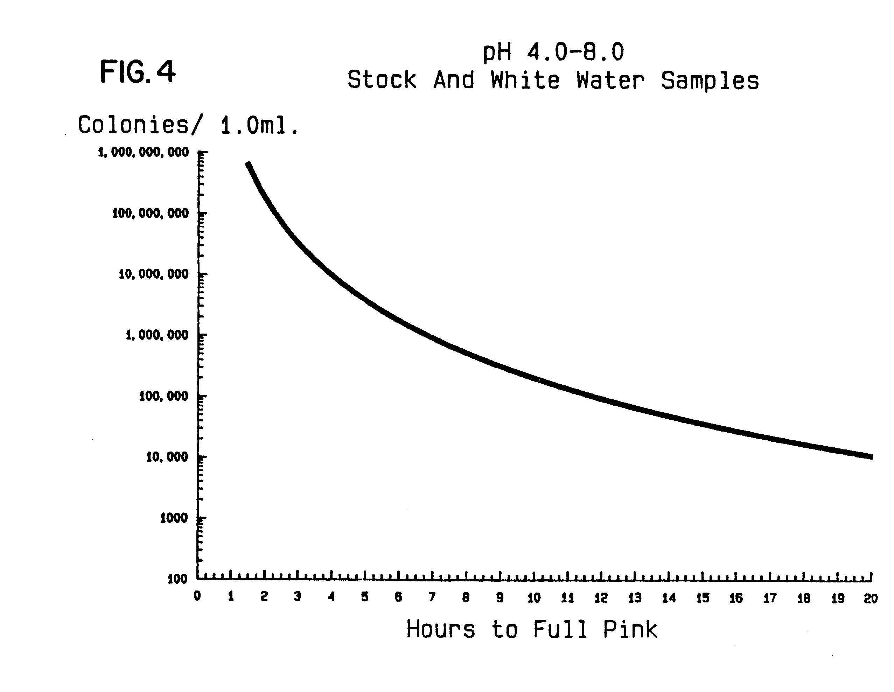

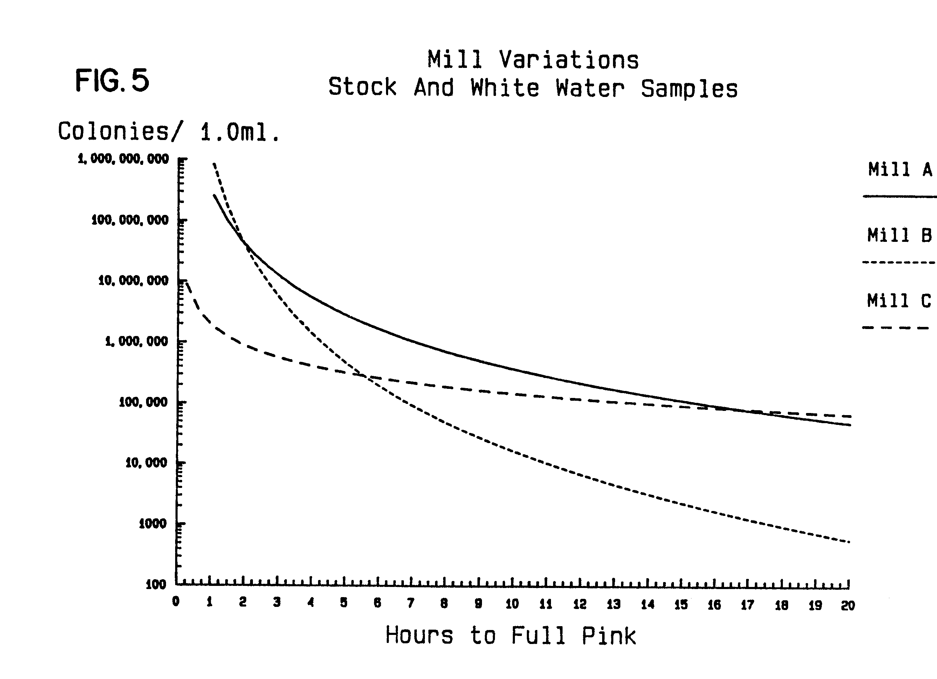

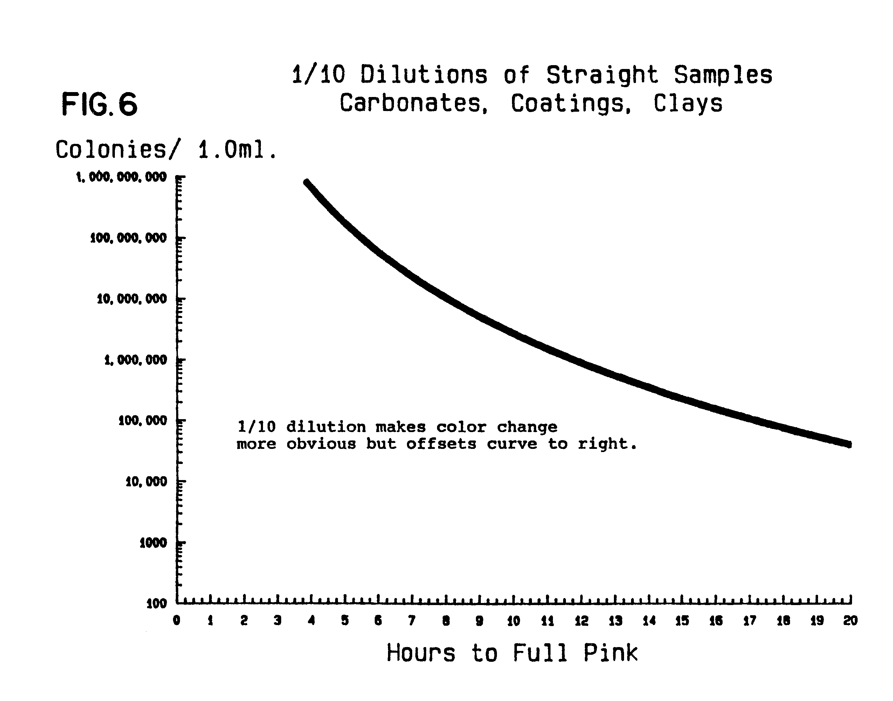

FIGURE 1 shows a vial containing a dried nutrient broth for use in the instant invention. The vial cap contains a dried indicator dye. FIGURES 2-6 show "Time to Color Change" vs. "Colonies/ml" curves.

Summary of the Invention

The instant invention relates to a method and device for rapid, simple and accurate enumeration of microorganisms, including bacteria, fungi and algae, in industrial process systems. The method relies on a dye system which, when exposed to actively respiring microorganisms, changes color at a rate directly proportional to the number of viable microorganisms present. The device comprises a vial which contains dried nutrients for enhanced microbial activity and a dried indicator dye. The dried nutrients and the dried inicator dye adhere to the bottom of the vial and the cap of the vial, respectively, and are thereby separated within the capped vial. A measured volume of aqueous sample suspected of containing microorganisms is then added to the glass container having dried nutrient and dye therein. The vial is capped and the contents mixed until the nutrient and dye dissolve in the sample. The vial is then incubated at 25-45°C and observed periodically until the color of its contents changes. The approximate microbial population is then determined via use of a standard plot showing the time needed for color change to occur on one axis and microorganism counts on the other axis.

Since microbial populations vary in composition and activity, standard plots are necessary for each sampling site to establish color change intervals corresponding to normal population levels at each site.

Utilizing the standard plots, a determination can quickly be made of the approximate concentration of microorganisms. The device can be used in a variety of industrial areas where microorganisms cause problems. For example, the instant devices may be used to monitor microorganism counts in pulp and papermill systems, industrial cooling water systems, metalworking fluid systems and other types of process systems. The method and device are especially useful in papermill applications where rapid, accurate and simple determination of organism levels is necessary to keep mills in operation.

The instant device and method may also have application as diagnositc or research tools in clinical or laboratory settings. There, the device might be used to enumerate microorganisms in body fluids such as urine or other laboratory specimens.

It is therefore one object of the instant invention to provide an enumeration device and method which are easy to use. The instant device and method require no involved manipulations, such as pipetting, mixing, agar melting, agar pouring, incubating and colony counting. To use the instant device, a sample suspected of containing microorganisms is added to a vial containing dried nutrients and dried indicator dye, which are separated within the vial. The contents are then mixed and incubated, and the device is periodically observed to determine when a color change occurs.

It is a further object of the instant invention to provide enumeration results rapidly. Enumeration of microorganisms is usually achieved within 8 hours, as compared to the traditional standard plate count method which requires 24 to 48 hours. If a problem exists where organism counts are excessively high, the instant method and device may indicate the presence of a problem in 1-2 hours. This quick indication enables plant personnel to quickly remedy the organism-related problem.

It is a further object of the instant invention to provide an enumeration device in a convenient form. The vial of the instant invention contains only dried, separated components, which eliminates spillage problems and lessens the chance for deterioration upon storage. Also, the device is small and easily stored.

It is still a further object of the instant invention to provide easy-to-read, accurate enumeration results. The color change at the endpoint is readily apparent, even in the presence of heavy suspended solids such as those found, for example, in papermaking applications.

Other objects of this invention may become apparent to those persons skilled in the art. For this reason, the inventors note that the above-stated objects in no way limit the scope of this invention.

Detailed Description of the Invention

The instant invention is directed to a method of enumerating microorganisms in an aqueous system comprising the steps of:

(a) adding an aqueous sample from said aqueous system to a vial, wherein said vial has a cap and wherein said vial, when capped, contains a dried nutrient medium and a dried indicator dye which are separated within said vial;

(b) capping said vial with said cap and mixing said sample with said dried nutrient medium and dried indicator dye, thereby dissolving said dried nutrient medium and said dried indicator dye into said sample;

(c) incubating said vial at 25-45°C;

(d) observing said vial periodically until a color change occurs due to a reaction of said dye with respiring microorganisms and noting the time required for said color change to occur; and

(e) comparing said time required for said color change to occur to a standard plot which correlates said time required for color change to occur on a first axis and microorganism counts on a second axis, thereby quickly enumerting microorganisms present in said sample.

The instant invention is also directed to a device for determining microbial counts comprising a vial, a cap for said vial, a dried nutrient broth and a dried indicator dye.

Any vial can be used. Preferred vials are glass, most preferably borosilicate glass. The size of the vial is not critical. However, the preferred vial capacity should be at least 15 ml. Excess volume is beneficial for mixing purposes. preferred vial is cylindrical in shape, about 2-3 inches high and about 1/2-1 inch in diameter. The vials should also be cappable. While any capping means can be used, it is preferred that the vials be threaded at the top so that correspondingly threaded caps may be screwed onto the vials, thereby closing them to the atmosphere. It is also preferred that the vial caps contain a vinylite-coated paper liner to facilitate addition of the dye to the inner surface of the cap, and subsequent dissolution of the dye into the sample.

The vials must contain a dried nutrient broth and a dried indicator dye. The dye and the broth must be in dried form, because the inventors have found that aqueous solutions containing the broth and dye are unstable. Also, contact between the nutrient broth and the dye should be avoided prior to use, as they may react even if in dried forms. Separation is preferably facilitated by adding an aqueous nutrient broth to the vial, and then drying the broth completely. The dried broth adheres to the bottom of the vial. Similarly, an aqueous dye solution is added to the cap and dried completely. The dried dye adheres to the cap. Thus, the nutrient and the dye remain separated within the capped vial.

Any microbiological culture medium may be used as the nutrient. A microbiological culture medium is defined herein as any general purpose medium for the cultivation of microorganisms. Preferred media include the nutrient broth containing Peptone 190 (pancreatic digest of gelatin) and beef extract, available from Gibco Laboratories as Catalog No. M36000; tryptic soy broth, available from Gibco Laboratories as Catalogue No. M49800; brain heart infusion broth (modified), available from Gibco Laboratories as Catalogue No. M06800; and R-2A broth, containing Pepton 185 (casein animal tissue polypeptone) Peptone 5 (acid digest of casein), yeast extract, destrose soluble starch, sodium pyruvate, potassium phosphate (dibasic) and magnesium sulfate, available from Gibo Laboratories as Catalogue No. M40400. The most preferred is the M36000 broth (nutrient broth).

When a liquid broth is added to a vial, the vial must be placed into a forced convection oven for drying of the broth or the broth must be dried by some other means. Recommended drying temperatures range from 50 to 70°C. The vial should remain in the oven until the nutrient broth is hard and dry without stickiness. The appearance of cracks in the broth is a good indication of dryness. Preferably, the dried broth adheres to the bottom of the vial.

Sufficient nutrient should be added to the vial so that at least 2 g of dried nutrient are present per liter of sample. The preferred dosage is 4-12 g/1 of sample, and the most preferred dosage is 7-9 g nutrient/1 sample.

The vial must also contain a dried indicator dye. Any indicator dye can be used, for example, resazurin, 2,3,5-triphenyltetrazolium chloride (TTC), methyl red, phenol pink, trypan blue, bromophenyl blue, nigrosine, cresol red, eosine y and erythrosin B. The preferred dyes are TTC and resazurin, and the most preferred dye is resazurin, which is an oxidation-reduction indicator dye. The dye must be used in the vial in a dried form. This can be accomplished by any suitable means. For example, the dye can be placed onto an inert substrate, oven-dried and the dried dye/substrate combination placed into the vial. Preferably, to insure that the nutrient and dye remain separated, the dye is placed into the vial cap and the cap/dye combination is oven dried. The dried dye adheres to the cap liner, and when the cap is screwed onto the vial, both the dye and the nutrient broth, in dried forms, will be contained in the vial.

Sufficient dye should be added to the vial (or cap) so as to provide a dye concentration in the sample of from about 0.001 to 5.0 mg/ml. The preferred concentration is about 0.01 to 0.1 mg dye/ml sample, and the most preferred dye concentration is about 0.02 to about 0.03 mg/ml of sample. Dye concentrations above 0.5 mg/ml should be avoided, as the dye may be toxic to the microorganisms being enumerated.

Ideally, the dye solution is placed on the paper liner of a screw cap. The dye should not be allowed to contact the side of the cap or spread beyond the center of the cap liner. The cap can then be placed in a forced air convection oven and heated until the dye is completely dry. The oven should not be heated above 65°C, or other temperature which may cause the cap to soften or melt. After the vial containing dried broth and the cap containing dried dye have cooled to room temperature, the cap is placed on the vial and tightened. This arrangement constitutes the claimed device for quantifying microorganism counts, as shown in FIGURE 1.

To use the claimed device, a liquid sample suspected of containing microorganisms is collected from an appropriate site. Approximately 10 ml of the sample is added to a vial containing dried dye (resazurin) and dried nutrient (M36000). It is preferred that each vial be marked to show its 10 ml mark. The vial is capped and then inverted several times to mix and dissolve the dried contents into the sample. The vial is then incubated at between 25 and 45°C, preferably 37-42°C. The operator should make certain that all dreid ingredients dissolve during the early stages of incubation.

For resazurin, the operator simply observes the vial hourly until the color changes from blue (or purple) to "shocking" pink. This means that the resazurin has been reduced from its blue oxidized state to a "shocking" pink reduced form known as resorufin. If the solution changes in color from pink to clear, the reaction has gone beyond the endpoint. Prior to each observation, the vial should be inverted at least once to obtain a uniform color.

To determine the approximate microbial population, the operator refers to a previously prepared standard plot showing "time for color conversion" on a first axis versus "colonies per 1.0 ml" on a second axis. Preferably, "time for color conversion" is on the "x" axis and "colonies per 1.0 ml" on the "y" axis. Then, by locating the hours to conversion on the "x" axis, the operator can read the colonies count from the "y" axis.

Obviously, the standard graph is prepared in advance of the test. The parameters used in the test described above are not critical. Thus, more or less than 10 ml of sample can be used, any amount of nutrient can be used, and any non-toxic dye concentration can be used. Also, any incubation temperature between 25 and 45°C can be used. The inventors believe that such modifications of the test procedure are well within the reach of a skilled artisan. However, it is imperative that the same parameters be used to prepare the origianl plot as are used with the claimed enumeration device in the field. Thus, the nutrient dosage, dye concentration and incubation temperature used when establishing the standard curve should be the same as when the device is used for field purposes.

The time required for the obvious color change (from blue to pink for resazurin) to occur is correlated to standard plate counts. The inventors have found, through laboratory testing, that the time for a sample to cause a color change is dependent solely upon the biological populations in the original sample, the incubation temperature, the nutrient dosage and dye concentration. While populations in the range of 100 organisms/ml to 1,000,000,0000/ml have been studied, the scope of these studies is in no way intended to limit the instant method.

Using the novel method and device disclosed herein, excessive biological populations can be detected in as little as 1 hour. This allows an operator to take corrective action before the effects of potential problems are translated into lost production, downtime or spoiled raw material.

The test method is effective and consistent despite wide variations in incubation temperatures or pH environments. See FIGURES 2-4. While the optimum incubation temperature is 37-42°C, the test has utility even when the incubation occurs in a shirt pocket. A variety of sample types respond, including wood pulp fiber suspensions, filler slurries and wood starch slurries.

Because different species of microorganisms may react differently in a test, a comparison with standard plate counts should be made for each sample point to improve accuracy. The test is intended to provide a rapid alert to high levels of biological activity. When systems containing low levels of biological activity are studied, it will take several hours for the color change to occur. If a sample produces an unusually rapid color change, an excessive biological upset in the system is indicated. This enables the cause of the upset to be investigated and corrective action to be taken before there is an economic impact upon the system.

The key to the instant invention is that it allows incompatible components (indicator dye and nutrient) to be held in a convenient way so that microbial populations can be quickly and accurately determined. The nutrient is required to expedite microbial growth, and the dye is used to enumerate the microbials present.

Examples

The following examples are not intended to in any way limit the scope of the instant invention.

Example 1 -- Preparation of Vials:

Vials were prepared by dispensing 0.5 ml of a 20 strength (160 g/l) sterile nutrient broth (Catalogue No. M36000), available from Gibco Laboratories, into 2-3/4" high x 3/4" diameter cylindrical borosilicate glass vials. The vials were then placed in a forced-air convection oven set for 55-65°C, where they remained until the nutrient broth was hard and dry, without stickiness. Sufficient drying was indicated by the appearance of cracks on the surface of the nutrient broth, and occured in about 6-10 hours, depending on the volume of the oven occupied by trays of drying vials. The dried nutrient adhered to the bottom of the vial.

Caps were prepared by dispensing 0.05 ml of a resazurin stock solution (0.5% resazurin dissolved in 0.01% NaOH solution) in the center of vinylite/paper liners of the vials' screw caps. The caps were then placed in a forced-air convection oven for about 1.5 hours, thereby drying the dye. The dried dye adhered to the cap liners. The oven temperature was set at 55-65°C.

After cooling, the caps were screwed on to the vials, forming the claimed enumeration device.

Example 2:

Samples were collected from various paper mill systems. Samples consisted primarily of mixtures of wood pulp fibers, water and various additives, such as clay, titanium oxide, aluminum sulfate and rosin. Some additives, such as starch and clay, were also sampled. 10 mls of the various samples were then added to the vials of Example 1. The vials were then capped, which resulted in each vial containing 10 ml of sample, 80 mg of nutrient and 0.25 mg of dye.

After tightening the caps, the vials were inverted several times to mix the contents. The vials were then incubated at approximately 37°C and the vials were observed to insure that the dye and nutrient solubilized. The vials were observed hourly until color changes occured. The times required for the vials to change to "shocking" pink were recorded.

While the vials were being set up, standard plate counts were also performed on the collected paper mill samples. After 48 hours of incubation, the number of colonies/ml of sample was determined. By plotting the time required for a sample to effect the characteristic color change versus plate count values for the respective samples, a standard curve was established. Once a curve had been established, the approximate number of microorganisms in similar samples could be determined, without further need of the standard plate count, by operation of the vials, observing the time required for color change, and then referring to the standard curve.

These curves appear herein as FIGURES 2-6.

FIGURE 2 shows the effect of incubation temperatures using a mixed culture of laboratory microorganisms. These organisms were: Aerobacter aerogenes (IPC No. 500), Bacillus mycoides (IPC No. 509), Pseudomonas aerugmosa (ATCC No. 10145). The organisms were grown at 37°C in 1 or 2 strength nutrient broth (M36000 broth). Overnight broth cultures were added directly to the vials, mixed, and incubated at the various temperatures shown. Broth cultures were also serially diluted at 10-fold intervals in sterile phosphate buffered water (Butterfield's buffer purchased from Gibco Laboratories, Catalogue No. T3550) and the dilutions were subsequently added to the vials, mixed, and incubated. The number of microorganisms in each dilution was quantified using standard plate counts.

FIGURE 2 plots the time, in hours, for a particular dilution to reach full pink versus the number of microorganisms in the dilution expressed as colonies/1.0 ml of diluted sample.

FIGURE 3 shows the effect of incubation temperatures on thick stock samples collected from an operating paper mill. The stock consisted of water, additives and fiber.

FIGURE 4 shows the effect of pH on stock and white water samples.

FIGURE 5 shows stock and white water curves for various mills, designated as mills A, B and C.

FIGURE 6 shows the effect of 1/10 dilutions of straight samples of various paper mill additives, including carbonate slurries, coating slurries and clay slurries.

## Claims

1. A method of enumerating microorganisms in an aqueous system suspected of containing microorganisms comprising the steps of:

(a) adding an aqueous sample from said aqueous system to a vial, wherein said vial has a cap and wherein said vial contains a dried nutrient medium and a dried indicator dye which are separated;

(b) capping said vial with said cap and mixing said sample with said dried nutrient medium and dried indicator dye, thereby dissolving said dried nutrient medium and indicator dye into said sample;

(c) incubating said vial at 25-45°C;

(d) observing said vial periodically until a color change occurs due to reaction of said dye with respiring microorganisms and noting time required for said color change to occur; and

(e) comparing said time required for the color change to occur to a standard plot which correlates said time required for color change to occur on a first axis and microorganism counts on a second axis, thereby quickly enumerating microorganisms present in said sample.

2. The method of Claim 1, wherein said nutrient medium is selected from the group consisting of a nutrient broth containing pancreatic digest of gelatin and beef extract; tryptic soy broth; brain heart infusion broth; and a broth containing casein animal tissue polypeptone, acid digest of casein, yeast extract, dextrose-soluble starch, sodium pyruvate, potassium phosphate (dibasic) and magnesium sulfate.

3. The method of Claim 1, wherein said indicator dye is selected from the group consisting of resazurin, 2,3,5-triphenyltetrazolium chloride, methyl red, phenol pink, trypan blue, bromophenyl blue, nigrosine, cresol red, eosine y and erythrosin B.

4. The method of Claim 3, wherein said dye is selected from the group consisting of resazurin and 2,3,5-triphenyltetrazolium chloride.

5. The method of Claim 1, wherein said nutrient medium is added to produce a dosage of at least 2 g dried nutrient per liter of sample and wherein said dye is added to produce a concentration of about 0.001 to 0.5 mg dye per ml of sample.

6. The method of Claim 5, wherein said dye adheres to said cap and wherein said nutrient medium adheres to said vial.

7. A device for determining microbial counts in aqueous systems, comprising a glass vial, a cap for said vial, a dried nutrient medium and a dried indicator dye, wherein said nutrient medium and dye remain separated.

8. The device of Claim 7, wherein said medium is a nutrient broth containing pancreatic digest of gelatin and beef extract.

9. The device of Calim 8, wherein said dye is resazurin.

10. The device of Claim 8, wherein said dye is in said cap and said medium is in said vial.

0244148

# FIG.1

Vial

Dried
Nutrient

Cap

Dried Dye
(Inside Cap)

**FIG. 2**

## Effects of Incubation Temperature
## With Laboratory Cultures

Colonies/ 1.0ml.

Hours to Full Pink

30 C

34 C

37 C

40 C

42 C

44 C

FIG. 3

Effects of Incubation Temperature
Thick Stock Samples

Colonies/ 1.0ml.

37C

40C

Hours to Full Pink

FIG.4

pH 4.0-8.0
Stock And White Water Samples

Colonies/ 1.0ml.

Hours to Full Pink

FIG.5

Mill Variations
Stock And White Water Samples

Colonies/ 1.0ml.

Mill A

Mill B

Mill C

Hours to Full Pink

# FIG. 6

**1/10 Dilutions of Straight Samples
Carbonates, Coatings, Clays**

Colonies/ 1.0ml.

1/10 dilution makes color change
more obvious but offsets curve to right.

Hours to Full Pink

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | US-A-3 415 718 (A.C.G. FORKMAN et al.) <br> * Whole document * | 1-3 | C 12 Q   1/06 |
| Y | US-A-3 313 712 (M.E. GEORGE) <br><br> * Claim 1; column 3, lines 1-14; figures * | 1,6,7, 10 | |
| Y | US-A-4 239 746 (D.I. BARTOS) <br><br> * Figure 2; column 11, lines 48-56 * | 1,6,7, 10 | |
| Y | CHEMICAL ABSTRACTS, vol. 83, no. 20, 17th November 1975, page 98, abstract no. 165836d, Columbus, Ohio, US; S.C. NANDY: " Rapid colorimetric method for the determination of bacterial population in tannery liquors and in raw hides and skins", & LEATHER SCI. (MADRAS) 1975, 22(5), 121-8 <br> * Abstract * | 1,6,7, 10 | TECHNICAL FIELDS SEARCHED (Int. Cl 4) <br><br> C 12 Q   1/00 |
| A | PATENT ABSTRACTS OF JAPAN, vol. 5, no. 94 (C-59)[766], 19th June 1981; & JP-A-56 35 996 (YOSHINOBU MITSUI) 08-04-1981 <br> * Abstract * | 3 | |

-/-

The present search report has been drawn up for all claims

| Place of search | Date of completion of the search | Examiner |
|---|---|---|
| THE HAGUE | 24-07-1987 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| | DOCUMENTS CONSIDERED TO BE RELEVANT | | Page 2 |
|---|---|---|---|
| **Category** | **Citation of document with indication, where appropriate, of relevant passages** | **Relevant to claim** | **CLASSIFICATION OF THE APPLICATION (Int. Cl.4)** |
| A | US-A-4 140 489  (S.Y. LEE)<br>* Figures; claims * | 1-10 | |
| | --- | | |
| A | FR-A-2 504 552  (S. KOLEHMAINEN et al.)<br>* Claims 3-8 * | 1 | |
| | ----- | | |

**TECHNICAL FIELDS SEARCHED (Int. Cl.4)**

The present search report has been drawn up for all claims

| Place of search | Date of completion of the search | Examiner |
|---|---|---|
| THE HAGUE | 24-07-1987 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82